**Europäisches Patentamt**

**European Patent Office**

**Office européen des brevets**

(19)

(11) Veröffentlichungsnummer: **0 035 788**

**A1**

(12) # EUROPÄISCHE PATENTANMELDUNG

(21) Anmeldenummer: **81101748.2**

(22) Anmeldetag: **10.03.81**

(51) Int. Cl.³: **G 01 N 33/18**
**G 01 N 15/06**

(30) Priorität: **10.03.80 DE 3009130**

(43) Veröffentlichungstag der Anmeldung:
**16.09.81 Patentblatt 81/37**

(84) Benannte Vertragsstaaten:
**AT BE CH DE FR GB IT LI NL SE**

(71) Anmelder: **Conducta Gesellschaft für Mess- und Regeltechnik mbH & Co.**
**Dieselstrasse 24**
**D-7016 Gerlingen b. Stuttgart(DE)**

(71) Anmelder: **Hager + Elsässer GmbH**
**Ruppmannstrasse 26**
**D-7000 Stuttgart-Vaihingen(DE)**

(72) Erfinder: **Bach, Dieter**
**Lilienweg 43**
**D-7014 Kornwestheim(DE)**

(72) Erfinder: **Jäckle, Heiner G.**
**Rotenwaldstrasse 179**
**D-7000 Stuttgart 1(DE)**

(72) Erfinder: **Marquardt, Kurt**
**Eschelbacher Weg 33**
**D-7031 Holzgerlingen(DE)**

(74) Vertreter: **Liedl, Gerhard et al,**
**Patentanwälte Liedl, Nöth, Zeitler Steinsdorfstrasse 21 - 22**
**D-8000 München 22(DE)**

(54) **Verfahren und Vorrichtung zur Bestimmung des Kolloidanteiles bzw. -indexes in Flüssigkeiten.**

(57) Der Kolloidanteil von Flüssigkeiten, insbesondere Wasser, z.B. bei der Aufbereitung von Wasser, wird ermittelt, in dem ein Teilstrom der Flüssigkeit durch eine Abzweigleitung (15) geführt und durch ein Messfilter (11) unter Aufwendung von Druck hindurchgeleitet wird. Die Änderung der durch das Messfilter (11) hindurchströmenden Flüssigkeitsmenge wird ermittelt.

./...

FIG.1

– 1 –

Verfahren und Vorrichtung zur Bestimmung
des Kolloidanteiles bzw. -indexes in Flüssigkeiten

Die Erfindung betrifft ein Verfahren zur Bestimmung des Kolloidanteils bzw. -indexes in Flüssigkeiten, insbesondere Wasser, z. B. bei der Aufbereitung von Wasser, bei dem aus der aufzubereitenden bzw. aufbereiteten Flüssigkeit eine bestimmte Menge zu messender Flüssigkeit (Meßflüssigkeit) durch ein Meßfilter unter Aufwendung von Druck hindurchgeleitet wird, sowie eine Vorrichtung zur Durchführung dieses Verfahrens. Ein derartiges Verfahren und eine derartige Vorrichtung lassen sich verwenden bei der Überwachung von

- Wirkungsgrad der Vorreinigungsstufe vor einer Umkehrosmoseanlage
- Überwachung von Umkehrosmoseanlagen
- Überwachung der Reinstwasserqualität nach Feinstfiltern
- Überwachung von Filteranlagen, z. B. Sand- oder Mehrschichtfilter, in der Wasseraufbereitung, anstelle von Trübungsmessung

E 112

- Überwachung von Kesselspeisewasser und Kondensat
- Reinstwasser-Überwachung im Hinblick auf Verunreinigungen durch Mikroorganismen.

Z. B. Wasserreinigungsanlagen nach dem Prinzip der Umkehrosmose gewinnen bei der Wasserentsalzung immer größere Bedeutung. Dabei wird das zu entsalzende Wasser mit hohem Druck durch Membranen gepreßt, die so beschaffen sind, daß die Wassermoleküle und die Inhaltsstoffe des Wassers getrennt werden. Dabei besteht jedoch die Gefahr, daß die Membranen durch besonders geartete Inhaltsstoffe des Wassers verblockt werden. Diese Verblockung äußert sich in Permeabilitätsverlusten, die irreversibel sind. Dies kann zum vollständigen Ausfall der teuren Umkehr-Osmose-Membranen führen.

Aufgabe der Erfindung ist es daher, ein Verfahren und eine Vorrichtung nach den Oberbegriffen der Ansprüche 1 und 3 dahingehend zu verbessern, daß auch während der Durchführung des Flüssigkeits- bzw. Wasseraufbereitungsprozesses, beispielsweise während der Entsalzung des Wassers, eine kontinuierliche Überwachung der zu behandelnden Flüssigkeit auf das Vorhandensein membranverschlackender Substanzen möglich ist.

Diese Aufgabe wird verfahrensmäßig durch die im Kennzeichen des Anspruchs 1 und vorrichtungsmäßig durch die im Kennzeichen des Anspruchs 3 angegebenen Merkmale gelöst.

Bei der Erfindung läßt sich während oder nach bzw. innerhalb von Teilschritten der Aufbereitung die Flüssigkeit (Meßflüssigkeit) in einer Bypaß-Leitung abzweigen und die Änderung der durch das

E 112

Meßfilter hindurchströmenden Flüssigkeitsmenge, bezogen auf die Zeit ermitteln und als Maß bei der Bestimmung des Kolloidanteils bzw. -indexes verwenden. Die bei derartigen diskontinuierlichen Meßverfahren ermittelten Werte werden als Silting-Index, Fouling-Index oder Kolloid-Index bezeichnet.

Dabei ist es möglich, während mehrerer aufeinanderfolgender Verfahrensschritte die Zeit zu bestimmen, während welcher jeweils bestimmte Volumina der aus dem Aufbereitungs-, insbesondere Reinigungsprozeß abgezweigten Meßflüssigkeit durch das Meßfilter hindurchgeströmt sind, oder die Volumina zu bestimmen, welche innerhalb vorbestimmter Zeiträume durch das Meßfilter gelaufen sind. Auch kann kontinuierlich die Durchflußänderung ermittelt werden.

In vorteilhafter Weise können die bislang zeitaufwendigen Handmessungen zur Ermittlung der gewünschten Meßgrößen mit Hilfe einer automatischen, kontinuierlichen oder quasikontinuierlichen Messung durchgeführt werden.

Die Vorrichtung, mit der sich das erfindungsgemäße Verfahren durchführen läßt, besitzt einen Sammel- und Meßbehälter, in welchem das durch das Meßfilter hindurchgepreßte Wasser aufgefangen wird. Dabei wird das durch das Meßfilter hindurchgepreßte Wasser mittels eines Rohres, das bis in die Nähe des Bodens des Sammel- und Meßbehälters reicht, geleitet. Auf diese Weise läßt sich eine ruhige Wasseroberfläche und damit eine genaue Ermittlung des im Sammel- und Meßbehälter befindlichen Wassers erzielen. Außerdem ist es möglich, das während der Testlaufzeit durch das Meßfilter hindurchgepreßte Wasser in unmittelbare Nä-

E 112

he zum Auslauf des Sammel- und Meßbehälters an dessen Boden definiert zu bringen.

Außerdem kann in bevorzugter Weise die Druckkammer, welche unmittelbar über dem Meßfilter liegt und in welcher die unter Druck stehende Meßflüssigkeit beim Hindurchpressen durch das Meßfilter sich befindet, so bemessen sein, daß in dieser Druckkammer entstehende Luftblasen nach oben entweichen können, so daß die Meßfilteroberfläche frei von Luftblasen ist. Die Druckkammerhöhe ist daher größer bemessen als der größtmögliche Durchmesser von Luftblasen. Bevorzugt hat die Druckkammer eine Höhe von etwa 10 mm.

In vorteilhafter Weise läßt sich während des Aufbereitungsprozesses, z. B. Reinigungsprozesses der Flüssigkeit, insbesondere vor Eintritt in die Membranentsalzungseinheit, eine Überwachung des Prozeßablaufes gewinnen. Insbesondere kann das Aufbereitungsergebnis einzelner Prozeßstufen ermittelt werden. Auch lassen sich Teile der Aufbereitungsanlagen dadurch schützen, daß der Anteil schädlicher Stoffe in der Flüssigkeit bestimmt werden kann. Insbesondere bei der Aufbereitung des Wassers läßt sich ständig ein Aufschluß über die Gefährlichkeit hinsichtlich einer irreversiblen Verblockung der Membran, welche beim Aufbereitungsprozeß zum Einsatz kommt, erzielen. Man gewinnt daher sowohl einen aktiven Schutz für die beim Aufbereitungsprozeß zum Einsatz kommenden Anlagenteile, z. B. von Umkehr-Osmose-Membranen, als auch eine Prozeßsteuermöglichkeit von eventuell erforderlichen Vor-, Teil- oder Endreinigungsstufen.

In den beiliegenden Figuren ist ein Ausführungsbeispiel der Erfindung dargestellt. Anhand dieser Figuren soll die Erfindung noch näher erläutert werden. Es zeigt:

Fig. 1    eine Meßanordnung im Vertikalschnitt und

Fig. 2    eine Anordnung zur Führung des Meßfilters.

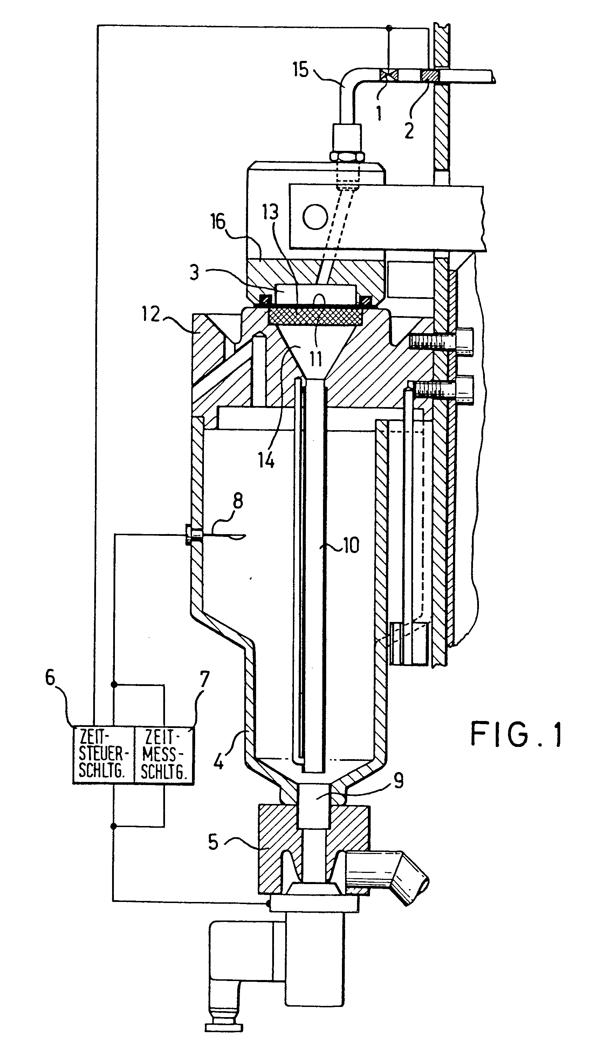

Die Fig. 1 zeigt eine Meßvorrichtung, die einen Sammel- und Meßbehälter 4 aufweist. Dieser Behälter ist abgedeckt mittels eines abdeckenden Blockes 12, der ein Stützsieb 13 aufnimmt, auf welchem ein Meßfilter 11 gelagert ist. Unterhalb des Stützsiebes 13 ist eine trichterförmige Ausnehmung 14 vorgesehen, deren unten zulaufendes Ende in ein Füllrohr 10 einmündet, welches seinerseits im Block 12 befestigt ist.

Das Füllrohr 10 erstreckt sich bis in die Nähe des Bodens bzw. Auslaßendes 9 des Sammel- und Meßbehälters 4. Am Auslaßende 9 ist ein Auslaßventil 5 vorgesehen.

Die Meßflüssigkeit gelangt, gesteuert durch ein Einlaßventil 1, über einen Druckregler 2 durch eine Zuleitung 15 in einen Druckraum 3. Dieser Druckraum 3 befindet sich in einem Spannkopf 16, der flüssigkeitsdicht auf das Meßfilter 11 aufgesetzt werden kann. Die Steuerung des Einlaßventils 1 und des Auslaßventils 5 erfolgt mit Hilfe einer Zeitsteuerschaltung 6. Die Zeitsteuerschaltung 6 arbeitet in Abhängigkeit eines höhenverstellbaren Niveauschalters 8, mit dem ein bestimmtes Füllvolumen im Sammel- und Meßbehälter 4 festgestellt werden kann. Mit Hilfe einer Zeitmeßschaltung 7, die ebenfalls an den Niveauschalter 8 angeschlossen

F 112

ist, können Zeitmessungen durchgeführt werden. Dabei kann die Zeit gemessen werden, welche benötigt wird, um ein bestimmtes Füllvolumen, das durch den Niveauschalter 8 voreinstellbar ist, zu erzielen. In bevorzugter Weise kann der Behälter 4 abgestuft sein, d.h. unterschiedliche Durchmesser besitzen, so daß auch geringere Füllvolumina mit ausreichender Genauigkeit über die Füllhöhe ermittelt werden können.

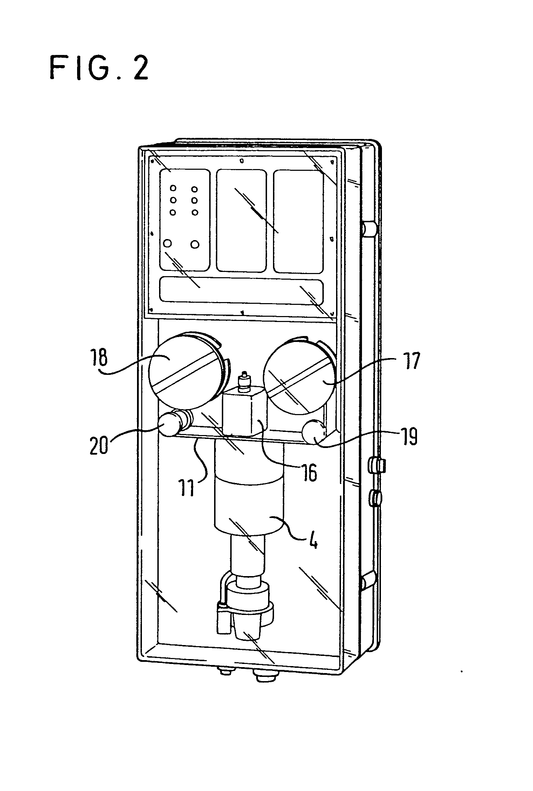

Aus der Fig. 2 ist zu ersehen, daß das Meßfilter 11 als Membranfilterband ausgebildet sein kann, das von einer Vorratsspule 18 über eine Umlenkrolle 20 durch den Druckraum, welcher vom Spannkopf 16 gebildet wird, hindurchgeführt wird. Der Spannkopf 16 spannt dabei das als Membranfilterband ausgebildete Meßfilter 11 ein und dichtet es ab und bildet dabei im Druckraum 3 eine definierte Filterfläche auf dem Filterband. Zum Aufsetzen des Spannkopfes 16 auf das Filterband und zum Abheben des Spannkopfes vom Filterband 11 dient ein nicht näher dargestellter Antrieb, beispielsweise ein Elektromotor. Das die Druckkammer 3 verlassende Filterband 11 wird über eine Umlenkrolle 19 geführt und auf einer Aufspulrolle 17 aufgewickelt.

Mit der dargestellten Vorrichtung läßt sich beispielsweise der folgende Verfahrensablauf durchführen:

Während eines ersten Verfahrensschrittes wird die Meßflüssigkeit beispielsweise mit 2 bar durch das Meßfilter 11 hindurchgepreßt, wobei das Auslaßventil 5 geschlossen bleibt. Es wird dabei die Sammelzeit $t_1$ gemessen, innerhalb welcher der Sammel- und Meßbehälter 4 bis zur Höheneinstellung des Niveauschalters 8 angefüllt ist. Anschließend wird durch Öffnen des Auslaßventils 5

E 112

der Behälter 4 entleert, und dann wird bei geöffnetem Auslaßventeil 5 Meßflüssigkeit für einen bestimmten Zeitraum (Testlaufzeit T) durch das Meßfilter 11 hindurchgedrückt, wobei ebenfalls ein Druck von 2 bar zu Anwendung kommen kann. Dabei werden in der Meßflüssigkeit enthaltene filtrierbare Substanzen zurückgehalten. Die Meßflüssigkeit entspricht der Flüssigkeit, welche im Aufbereitungszyklus, beispielsweise bei der Entsalzung von Wasser, den Umkehr-Osmose-Membranen zugeführt wird.

Nach Beendigung der Testlaufzeit, welche beispielsweise 5 oder 15 min betragen kann, wird das Auslaßventil 5 geschlossen, und es wird die Zeit gemessen, während welcher das Sammel- und Meßgerät 4 bis in die Höhe des Niveauschalters 8 wieder aufgefüllt wird. Diese Zeit wird als zweite Sammelzeit $t_2$ bezeichnet. Als durch den Niveauschalter 8 voreinstellbares Volumen können beispielsweise 100 oder 500 ml eingestellt sein. Der Kolloidanteil bzw. -index (KI) ergibt sich nach der unten stehenden Formel, wobei hierzu eine nicht näher dargestellte Auswerteschaltung verwendet werden kann.

$$KI = \frac{(1 - \dfrac{t_1}{t_2}) \cdot 100}{T}$$

Diese Formel ist auch für die Definition des Verblockungsindexes aus dem "Technical Bulletin" der Firma DuPont Nr. 491 vom 1. März 1976 bekannt.

Insofern läßt sich hierdurch eine genaue Angabe über den Kolloidanteil bzw. -index während der Aufbereitung der Flüssigkeit ständig erzielen.

E 112

- 1 -

Patentansprüche:

1.    Verfahren zur Bestimmung des Kolloidanteiles bzw. -indexes in Flüssigkeiten, insbesondere Wasser, z.B. bei der Aufbereitung von Wasser, bei dem zum Messen eine bestimmte Menge der Flüssigkeit (Meßflüssigkeit) während oder nach bzw. innerhalb von Teilschritten der Aufbereitung in einer Abzweigleitung abgezweigt, durch ein Meßfilter unter Aufwendung von Druck hindurchgeleitet wird und die Änderung der durch das Meßfilter hindurchströmenden Flüssigkeitsmenge, bezogen auf die Zeit, ermittelt und als Maß bei der Bestimmung des Kolloidanteils bzw. -indexes verwendet wird, d a d u r c h   g e k e n n z e i c h n e t ,   daß

-    entweder in mehreren aufeinanderfolgenden Schritten die jeweiligen Volumina der aus dem Aufbereitungsprozeß abgezweigten Meßflüssigkeit, welche jeweils innerhalb vorbestimmter Zeiträume durch das Meßfilter gelaufen sind, ermittelt werden,

-    oder die Änderung der durch das Meßfilter fließenden Flüssigkeit, bezogen auf die Zeit (Durchflußänderung) kontinuierlich ermittelt wird.

2.    Verfahren nach Anspruch 1, d a d u r c h   g e k e n n z e i c h n e t ,   daß die durch das Meßfilter filtrierte Flüssigkeit gesammelt wird, wobei die Volumenmessung und die automatische Steue-

E 112

rung der zeitweisen Bevorratung der filtrierten Flüssigkeit mittels einer höhenverstellbaren Niveausonde erfolgt.

3. Vorrichtung zur Bestimmung des Kolloidanteils bzw. -indexes in Flüssigkeiten, insbesondere Wasser, z. B. bei der Aufbereitung von Wasser, mit einer Druckeinrichtung, die eine auf die Meßmembran aufsetzbare Druckkammer aufweist und mittels der die Meßflüssigkeit durch ein Meßfilter hindurchgepreßt wird, einem unter dem Meßfilter angeordneten Sammel- und Meßbehälter für die durch das Filter gepreßte Flüssigkeit und Volumen- und Zeitmeßeinrichtungen zur Durchführung eines der Verfahren nach Anspruch 1 oder 2, d a d u r c h  g e k e n n z e i c h n e t , daß, gesteuert durch eine Zeitsteuerschaltung, ein Einlaßventil (1), durch welches über einen Druckregler (2) Meßflüssigkeit in die Druckkammer (3) fließt, geöffnet ist und ein am Auslaß des Sammel- und Meßgefäßes (4) angeordnetes Auslaßventil (5) geschlossen ist, das nach Ablauf einer bestimmten Zeit geöffnet wird und während einer bestimmten Testlaufzeit geöffnet bleibt, und daß schließlich nach Ablauf der Testlaufzeit das Auslaßventil (5) eine bestimmte Zeitdauer geschlossen ist und daß nach Ablauf dieser Zeitdauer das Einlaßventil (1) geschlossen wird.

4. Vorrichtung nach Anspruch 3, d a d u r c h  g e k e n n z e i c h - n e t , daß die Zeitsteuerschaltung (6) in Abhängigkeit von der Höheneinstellung eines höhenverstellbaren Niveauschalters (8) arbeitet, der ein elektrisches Signal abgibt, wenn die im Sammel- und Meßbehälter (4) ansteigende Meßflüssigkeit das Niveau des Schalters (8) erreicht hat.

E 112

5.    Vorrichtung nach Anspruch 3 oder 4, d a d u r c h   g e - k e n n z e i c h n e t,  daß bis in die Nähe des am Boden des Sammel- und Meßbehälters (4) befindlichen Flüssigkeitsauslasses (9) ein Füllrohr (10) sich erstreckt, durch welches die durch das Meßfilter gepreßte Meßflüssigkeit in den Sammel- und Meßbehälter eingebracht wird.

6.    Vorrichtung nach einem der Ansprüche 3 bis 5, d a d u r c h   g e k e n n z e i c h n e t,  daß die Höhe der Druckkammer (3) größer bemessen ist als der Durchmesser von in der im Druckraum befindlichen Flüssigkeit vorhandenen Luftblasen.

7.    Vorrichtung nach einem der Ansprüche 3 bis 6, d a d u r c h   g e k e n n z e i c h n e t,  daß der Sammel- und Meßbehälter (4) unterschiedlich abgestufte Durchmesser aufweist.

8.    Vorrichtung nach einem der Ansprüche 3 bis 7, d a d u r c h   g e k e n n z e i c h n e t,  daß in einem den Sammelbehälter (4) abdeckenden Block (12) ein Stützsieb (13) aufgenommen ist, durch welches das Meßfilter (11) abgestützt ist, daß unter dem Stützsieb (13) eine trichterförmige Ausnehmung (14) in den Block (12) eingeformt ist, die an ihrem unteren verengten Auslaßende in das im Block am oberen Ende befestigte Füllrohr (10) mündet.

9.    Vorrichtung nach einem der Ansprüche 3 bis 8, d a d u r c h   g e k e n n z e i c h n e t,  daß das Meßfilter (11) als Membranfilterband ausgebildet ist, das schrittweise von einer Vorratsrolle (18) durch die Druckkammer (3) zu einer Aufspulrolle (17) geführt ist.

E 112

FIG.1

FIG. 2

**Europäisches Patentamt**

## EUROPÄISCHER RECHERCHENBERICHT

| | EINSCHLÄGIGE DOKUMENTE | | | KLASSIFIKATION DER ANMELDUNG (Int. Cl.) |
|---|---|---|---|---|
| Kategorie | Kennzeichnung des Dokuments mit Angabe, soweit erforderlich, der maßgeblichen Teile | | betrifft Anspruch | |
| | <u>US - A - 3 138 015</u> (H.H. AVERY)<br>* Spalte 3, Zeile 11 bis Spalte 4, Zeile 2; Spalte 5, Zeilen 18-39; Figuren 1,2 *<br><br>-- | | 1,3,<br>8,9 | G 01 N 33/18<br>15/06 |
| | MESURES, März 1970,<br>Paris, FR<br>J.JOLLIVET: "Les filtres membranes auxiliaires de la mesure".<br>Seiten 65-69<br><br>* Seite 67, unten bis Seite 68, oben "Mesure de l'indice de colmatage" *<br><br>-- | | 1 | |
| A | <u>US - A - 3 985 020</u> (J.O. MOREAU)<br>* Spalte, 2, Zeile 40 bis Spalte 3, Zeile 33; Spalte 7, Zeilen 38-56; Figur 1 *<br><br>---- | | 3,8 | RECHERCHIERTE SACHGEBIETE (Int. Cl.)<br><br>G 01 N 33/18<br>15/00<br>15/06<br>11/00<br>11/02<br>11/04<br>11/06<br>B 01 D 37/00<br>37/04 |

KATEGORIE DER GENANNTEN DOKUMENTE

X: von besonderer Bedeutung

A: technologischer Hintergrund

O: nichtschriftliche Offenbarung

P. Zwischenliteratur

T der Erfindung zugrunde liegende Theorien oder Grundsatze

E kollidierende Anmeldung

D in der Anmeldung angeführtes Dokument

L aus andern Grunden angeführtes Dokument

& Mitglied der gleichen Patentfamilie, übereinstimmendes Dokument

Der vorliegende Recherchenbericht wurde für alle Patentansprüche erstellt

| Recherchenort | Abschlußdatum der Recherche | Prüfer |
|---|---|---|
| The Hague | 11-06-1981 | KEMPIN |

EPA form 1503.1  06.78